# EUROPEAN PATENT APPLICATION

(11) **EP 3 933 034 A1**
(43) Date of publication of application: **05.01.2022**
(21) Application number: 20763536.8
(22) Date of filing: 21.02.2020
(51) Int. Cl.: C12N 5/0775, C12N 5/0735, C12N 5/074, C12N 5/0789, C12N 5/0797, C12N 5/10, C12M 3/00

(54) **CELLULAR SPHEROID PRODUCTION METHOD**

(30) Priority: 25.02.2019 JP 2019032200
(71) Applicant: Nippon Shokubai Co., Ltd., Osaka-shi, Osaka 541-0043 (JP)
(72) Inventor: MAKINO, Tomomi, Suita-shi, Osaka 564-0034 (JP); SHIMA, Fumiaki, Suita-shi, Osaka 564-0034 (JP)
(74) Representative: Lederer & Keller Patentanwälte Partnerschaft mbB
(86) International application number: PCT/JP2020/007245
(87) International publication number: WO 2020/175410

(57) **Abstract**

Provided are a method for producing undifferentiated cell spheroids, the method comprising the step of culturing undifferentiated cells on a cell-adhesive surface of a cell culture sheet; a method for maintaining cell spheroids in an undifferentiated state, the method comprising the step of culturing undifferentiated cell spheroids on a cell-adhesive surface of a cell culture sheet; and a cell spheroid obtained by the method.

## Description

### TECHNICAL FIELD

The present invention relates to a method for producing cell spheroids. More particularly, the present invention relates to a method for producing undifferentiated cell spheroids, a method for maintaining the spheroids in an undifferentiated state, and a cell spheroid obtained by the method.

### BACKGROUND ART

In recent years, the use of undifferentiated stem cells such as iPS cells in regenerative medicine has been gaining momentum, and with a view to clinical application, there has been a lot of research on techniques for culturing cells in an undifferentiated state.

For example, Patent Literature 1 discloses a cell culture carrier having specific pores. Due to this structure, when undifferentiated stem cells such as ES cells and iPS cells are seeded on this carrier, some of the seeded cells outside wells can be guided into the inside of the wells by suction from the underside of the carrier and/or pressurization from the upper surface side of the carrier to facilitate efficient cell aggregation, thereby allowing the cells to proliferate in an undifferentiated state and to form spheroids.

In Non Patent Literature 1, spheroid formation is achieved by culturing embryonic stem cells seeded in wells of a V-bottom low-attachment cell culture plate.

Patent Literature 2 discloses efficient induction of reprogrammed somatic cells into iPS cells, which is achieved by seeding them in fibronectin-coated culture vessels and culturing them in serum-free medium without feeder cells. This disclosure shows that, under such culture conditions, the iPS cells can maintain their undifferentiated state and retain their pluripotency for a long period of time. For subsequent formation of embryoid bodies, low-attachment cell culture plates are used as in Non Patent Literature 1.

Patent Literature 3 discloses cell culture under a specific gravity environment, which enables iPS cells to proliferate and form spheroids and grow while keeping them in an undifferentiated state, even in the absence of feeder cells or coating agents.

### CITATION LIST

### Patent Literature

Patent Literature 1: JP-A 2017-212972
Patent Literature 2: JP-A 2015-123079
Patent Literature 3: JP 6421374

### Non Patent Literature

Non Patent Literature 1:
Cell Stem Cell 10, 771-785, June 14, 2012

### SUMMARY OF INVENTION

### TECHNICAL PROBLEM

The cell culture carrier described in Patent Literature 1 enables spheroid formation, but is an opaque porous product made by sintering ceramics, making it difficult to directly observe the inside of the vessel. In the vessels used in the Non Patent Literature 1 and Patent Literature 2, the culture surface is not adhesive, and only a single spheroid can be formed in a single well. This makes it difficult to obtain a large number of cells. In the method of Patent Literature 3, the creation of the gravity environment involves adjusting the rotational speed of a rotary bioreactor. This means that the method requires complicated operations and causes concerns about deterioration of cell functions due to rotational shear stress on cells. Therefore, all of these still have room for improvement, and further technological improvements are required.

An aspect of the present invention is intended to provide a novel method for producing undifferentiated cell spheroids, a novel method for maintaining the spheroids in an undifferentiated state, and an undifferentiated cell spheroid obtained by the method. Another aspect of the present invention is intended to provide a novel method for producing cell spheroids having a significantly higher integrin expression and an integrin-expressing cell spheroid obtained by the method.

### SOLUTION TO PROBLEM

In general, undifferentiated cells such as iPS cells need to be cultured on feeder cells or on substrates coated with biological components such as collagen. However, as a result of intensive study to achieve the above objectives, the present inventors found that culturing undifferentiated cells on a culture surface with a specific surface not only enables the production of undifferentiated cell spheroids, but also the maintenance of their undifferentiated state. Based on this finding, the present inventors completed one aspect of the present invention. The present inventors also found that culturing undifferentiated cells on a culture surface with a specific surface enables the production of cell spheroids having a significantly higher integrin expression than cell spheroids in suspension culture on a non-cell adhesive surface. Based on this finding, the present inventors completed another aspect of the present invention.

That is, the present invention relates to the following [1] to [14].
[1] A method for producing undifferentiated cell spheroids, the method comprising the step of culturing undifferentiated cells on a cell-adhesive surface of a cell culture sheet.
[2] A method for maintaining cell spheroids in an undifferentiated state, the method comprising the step of culturing undifferentiated cell spheroids on a cell-adhesive surface of a cell culture sheet.
[3] A method for producing integrin-expressing (integrin-containing) cell spheroids, the method comprising the step of culturing cells (cells that express or are capable of expressing an integrin, particularly undifferentiated cells) on a cell-adhesive surface of a cell culture sheet.
[4] The method according to any one of the above [1] to [3], wherein the cell-adhesive surface comprises a cell-adhesive substance.
[5] The method according to any one of the above [1] to [4], wherein the cell-adhesive surface comprises a polyimide resin.
[6] The method according to any one of the above [1] to [5], wherein the culturing is performed on a flat cell-adhesive surface.
[7] The method according to any one of the above [1] to [6], wherein the culturing is performed on a cell culture sheet having a plurality of recesses each having an opening of 2, 000 µm or less in diameter, wherein each recess has an inner circumferential face and a bottom face, wherein the inner circumferential face has a non-cell adhesive surface, and wherein the bottom face has a cell-adhesive surface.
[8] The method according to any one of the above [1] to [7], wherein the culturing is performed in the absence of feeder cells.
[9] The method according to any one of the above [1] to [8], wherein the cells are undifferentiated stem cells or progenitor cells.
[10] The method according to the above [9], wherein the stem cells are hematopoietic stem cells, mesenchymal stem cells, neural stem cells, tissue stem cells, embryonic stem cells, or pluripotent stem cells.
[11] A cell spheroid obtained by the method according to any one of the above [1] to [10].
[12] An undifferentiated cell spheroid having a relative mRNA expression level of an undifferentiation marker that is at least 3-fold higher than the corresponding level in a spheroid in suspension culture on a non-cell adhesive surface, as determined per 1 × 10⁵ cells.
[13] A cell spheroid having a protein expression level of an integrin that is at least 1.2-fold higher than the corresponding level in a spheroid in suspension culture on a non-cell adhesive surface.
[14] The spheroid according to any one of the above [11] to [13], wherein the spheroid has a diameter of 10 to 1,500 µm.

### ADVANTAGEOUS EFFECTS OF INVENTION

According to the present invention, spheroids that maintain their undifferentiated state can be produced easily and efficiently.

### BRIEF DESCRIPTION OF DRAWINGS

Fig. 1 is a schematic view of a cross-sectional structure of one embodiment of the cell culture sheet that can be used in the present invention.
Fig. 2 is a schematic view of a cross-sectional structure of one embodiment of the cell culture sheet that can be used in the present invention.
Fig. 3 is a schematic view of a cross-sectional structure of one embodiment of the cell culture sheet that can be used in the present invention.
Fig. 4 is a schematic view of cell culture in one embodiment of the cell culture sheet that can be used in the present invention.
Fig. 5 is a schematic view of cell culture in one embodiment of the cell culture sheet that can be used in the present invention.
Fig. 6 is a schematic view of a cross-sectional structure of one embodiment of the cell culture sheet that can be used in the present invention.
Fig. 7 shows images of embryoid bodies formed in Example 1. Scale bars indicate 200 µm.
Fig. 8 shows images of an embryoid body formed in Comparative Example 1. Scale bars indicate 200 µm.
Fig. 9 shows the relative expression level of an undifferentiation marker.
Fig. 10 is a photograph of one embodiment of the cell culture sheet or cell culture vessel that can be used in the present invention, taken from the bottom side of the recesses. The scale bar in Fig. 10a indicates 1 mm, and the scale bar in Fig. 10b indicates 5 cm.
Fig. 11 shows the protein expression levels of undifferentiation markers.
Fig. 12 shows the expression level of integrin αv.
Fig. 13 is a set of fluorescence micrographs of cell spheroids cultured using a cell culture sheet that can be used in the present invention. The scale bar indicates 100 µm.

### DESCRIPTION OF EMBODIMENTS

One aspect of the present invention provides a method for producing undifferentiated cell spheroids, the method comprising the step of culturing undifferentiated cells on a cell-adhesive surface of a cell culture sheet. The undifferentiated cell spheroid refers to a cell mass that can be induced to differentiate into desired cells in the presence of a cell growth factor, a differentiation inducer, etc. Another aspect of the present invention provides a method for producing integrin-expressing cell spheroids, the method comprising the step of culturing cells on a cell-adhesive surface of a cell culture sheet.

The "cell-adhesive surface" in the present invention is, for example, a surface on which cells adhere at certain points of adhesion when they settle on the surface in a solution used for culture. Alternatively, the cell-adhesive surface is a surface on which cells are immobilized at an adhesion strength that allows easy detachment of the cells by pipetting or other liquid flows. The cell-adhesive surface can be a surface on which cells adhere at a certain strength to form a steric or three-dimensional tissue structure, such as a layered structure or a spheroid, rather than a surface on which cells are two-dimensionally maintained and proliferated in an adherent state. Such a surface is presumed to allow moderate adhesion of cells or undifferentiated cells on the sheet and appropriate cell-cell interaction, thereby enabling close cell-to-cell communication and cell proliferation in an undifferentiated state, as well as spheroid (cell mass) formation (even formation of adherent spheroids, which can be detached without the use of enzymes or other chemicals and retain adhesive property after detachment). In addition, when cultured on such a cell-adhesive surface, cells receive some kind of stimuli that are different from those received under ordinary suspension culture conditions and thereby express high levels of integrins, which are factors that can affect cell migration, proliferation, differentiation, and survival. The spheroids having high integrin expression are unlikely to undergo cell death even in their inner part due to the action of the integrins. Such spheroids are presumed to contribute to tissue regeneration because they can efficiently adhere, extend, proliferate, and differentiate at a transplantation site. In addition, high expression of integrins is believed to strongly contribute to inhibition of stem cell differentiation, and when undifferentiated cells in culture have high integrin expression, the cells tend to maintain their undifferentiated state. However, this hypothesis is not intended to limit the present invention.

The cell-adhesive surface at least comprises a cell-adhesive substance.

The cell-adhesive substance is not particularly limited as long as the cells used for culture adhere to the substance or as long as the substance binds to cell surface molecules, such as proteins and sugar chains, present on the cell membrane of the cells used for culture. The cell-adhesive substance may be hydrophilic or hydrophobic. In view of cell adhesiveness, spheroid-forming efficiency, etc., hydrophilic (particularly, not ultra-hydrophilic) or hydrophobic (particularly, not ultra-hydrophobic) substances are preferred, and hydrophobic substances are more preferred. The cell adhesiveness of the cell-adhesive substance may be at a level that can prevent cells from accidentally jumping out of the recess.

Examples of the cell-adhesive substance include substances harvested from the living body and synthetic substances, for example, proteins (collagen, fibronectin, laminin, etc.) and synthetic resins (fluorine resins, polyimide resins, polysulfone, polyether sulfone, polydimethylsiloxane, a mixture thereof, etc.). In the case where synthetic resins are used as the cell-adhesive substance, an easy-to-handle cell culture sheet can be produced due to the strength and heat resistance of synthetic resins. Among synthetic resins, polyimide resins are preferred. The benefits of using polyimide resins are as follows: they are biocompatible; more homogeneous production of spheroids can be achieved; medium change can easily be done because polyimide resins have moderate cell-adhesiveness for various types of cells; spheroids in an adherent state can be obtained; and more homogeneous production of spheroids can be achieved. When nonbiological components such as polyimide resins are used in a cell culture sheet, spheroids obtained using the cell culture sheet can readily be applied to the fields including regenerative medicine and drug development.

The polyimide resin can be, for example, a polyimide resin having a structural unit represented by the formula (I) shown below. For efficient spheroid formation, a resin having a fluorine atom in the molecule is preferred, and a fluorine-containing polyimide (fluorine-containing polyimide resin) is more preferred. The polyimide resin used in the present invention can typically be obtained by imidization of a polyamic acid obtained by polymerization of one or more kinds of acid dianhydrides and one or more kinds of diamines. The polyimide resin may contain a polyamic acid as a partial chemical structure. The polyimide resin may be produced by any known method. For example, a two-step synthesis method can be used. Briefly, a polyamic acid is first synthesized as a precursor, and the polyamic acid is then converted into a polyimide. The polyamic acid used as a precursor of the polyimide resin may be a polyamic acid derivative. Examples of the polyamic acid derivative include polyamic acid salts, polyamic acid alkyl esters, polyamic acid amides, polyamic acid derivatives from bismethylidene pyromellitide, polyamic acid silyl esters, polyamic acid isoimides, etc. Examples of the polyimide include polyimides derived from a combination of an acid anhydride, such as pyromellitic acid dianhydride, biphenyl tetracarboxylic dianhydride, or benzophenone tetracarboxylic dianhydride, and a diamine, such as oxydiamine, p-phenylenediamine, m-phenylenediamine, or benzophenonediamine. Examples of the resin containing a fluorine atom include fluorine-containing polyimide resins having a structural unit represented by the formula (I) shown below, such as 4,4'-(hexafluoroisopropylidene)diphthalic anhydride (6FDA)/1,4-bis(aminophenoxy)benzene (TPEQ) copolymer, 6FDA/4,4'-oxydiphthalic anhydride (ODPA)/TPEQ copolymer, 4,4'-(4,4'-isopropylidenediphenoxy)diphthalic acid (BPADA)/2,2-bis[4-(4-aminophenoxy)phenyl]hexafluoropropane (HFBAPP), 6FDA/2,2-bis(4-(4-aminophenoxy)phenyl)propane (BAPP) copolymer, etc.; ethylene-tetrafluoroethylene copolymers; etc.

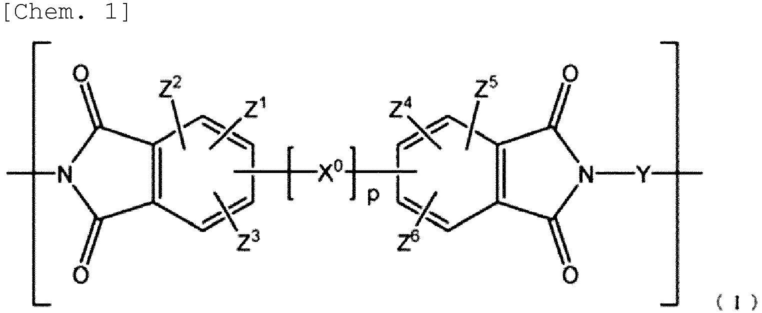

In the above formula (I), X⁰ represents an oxygen atom, a sulfur atom, a carbonyl group, a sulfonyl group, or a divalent organic group; Y represents a divalent organic group; Z¹, Z², Z³, Z⁴, Z⁵, and Z⁶ independently represent a hydrogen atom, a fluorine atom, a chlorine atom, a bromine atom, or an iodine atom; and p is 0 or 1. In the polyimide resin, the chemical structure represented by formula (I) may be different or identical for each structural unit of the resin. At least one of X⁰, Y, Z¹, Z², Z³, Z⁴, Z⁵, and Z⁶ preferably contains one or more fluorine atoms.

In the above formula (I), when p is 0, X⁰ may not be present (in other words, the benzene rings on the right and left sides are directly bound to each other). When p is 1, the benzene rings on the right and left sides are bound to each other via X⁰.

Specific examples of the divalent organic group represented by X⁰ include an alkylene group, an arylene group, an aryleneoxy group, an arylenethio group, etc. Also included are a fused cyclic divalent hydrocarbon group, a fused heterocyclic divalent hydrocarbon group, and a group that is derived from any of these hydrocarbon groups and contains an oxy or thio group. Among them, an alkylene group, an aryleneoxy group, and an arylenethio group are preferred, and an alkylene group and an aryleneoxy group are more preferred, and each of these groups may be substituted with a fluorine atom. The number of carbon atoms in the above alkylene group is, for example, 1 to 12, preferably 1 to 6.

The alkylene group substituted with a fluorine atom, which is an example of X⁰, may be, for example, -C(CF₃)₂-, -C(CF₃)₂-C(CF₃)₂-, etc. Among these examples of the alkylene group represented by X⁰, -C(CF₃)₂- is preferred.

The arylene group, which is another example of X⁰, may be, for example, any of the following groups.

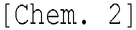

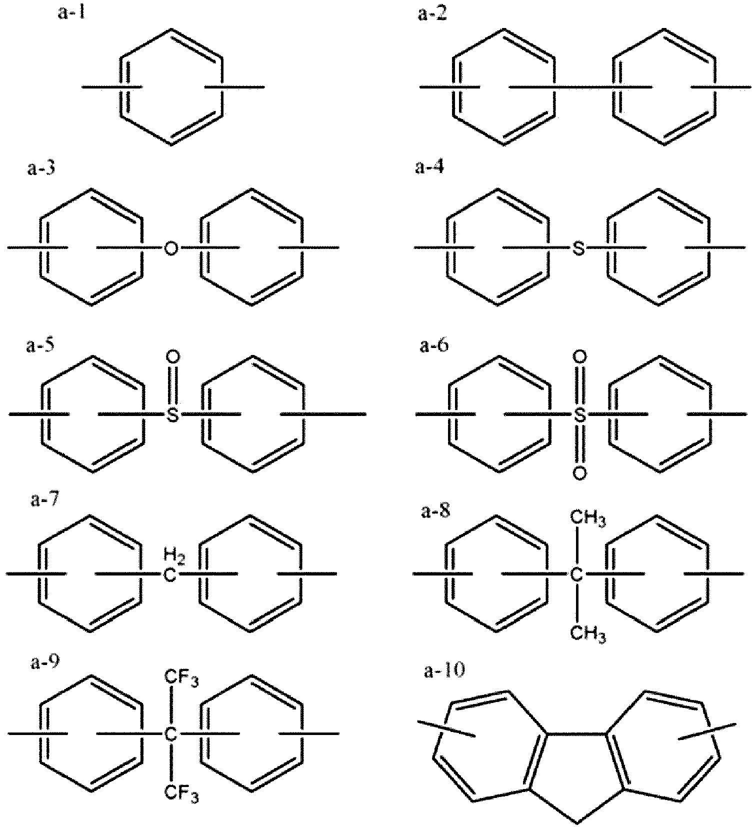

The aryleneoxy group, which is another example of X⁰, may be, for example, any of the following groups.

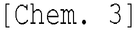

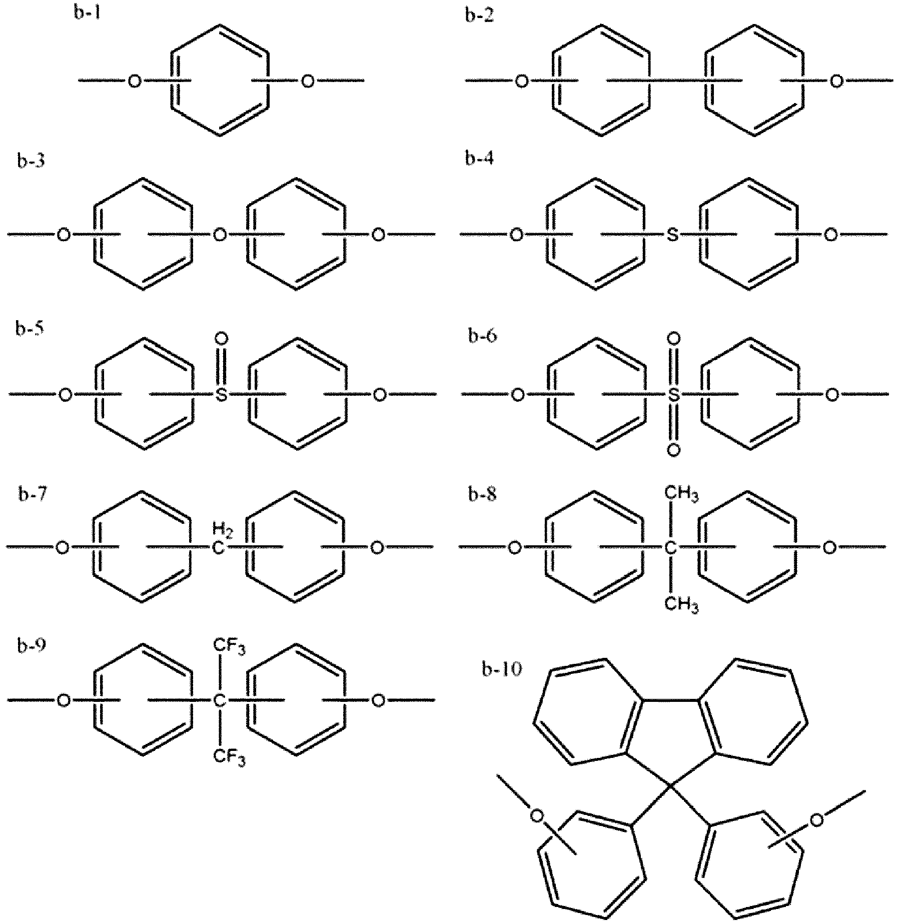

The arylenethio group, which is another example of X⁰, may be, for example, any of the following groups.

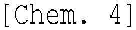

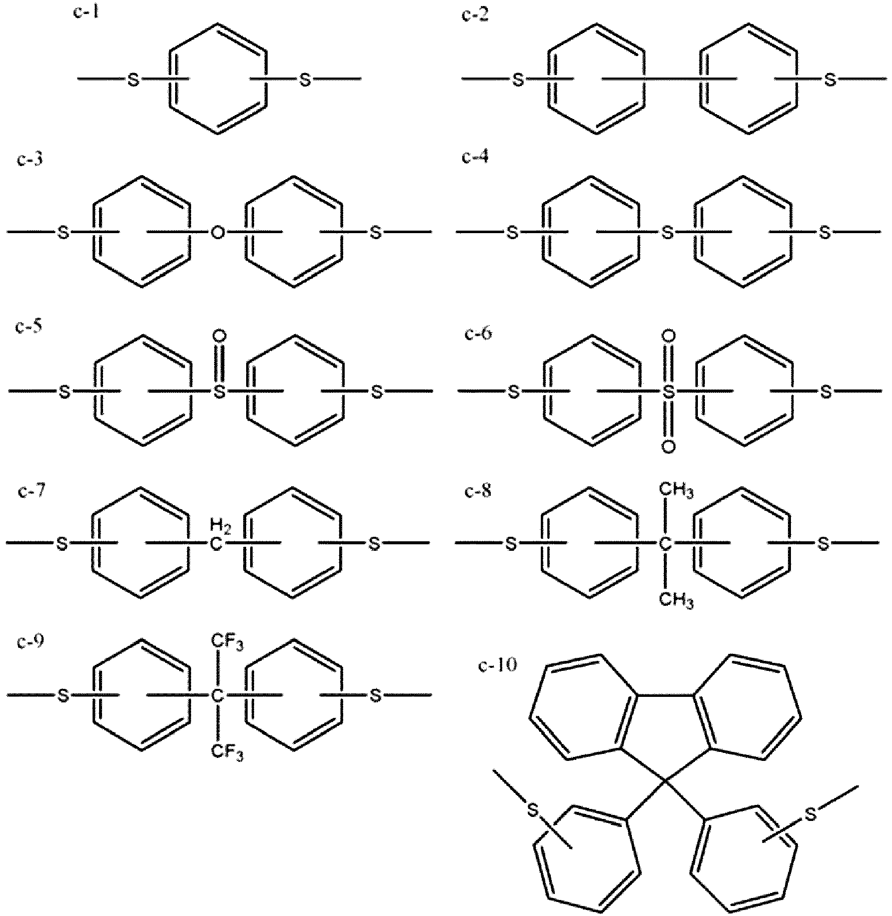

For efficient spheroid formation on a substrate, the divalent organic group represented by X⁰ may be selected from the group consisting of the above b-2 to b-10 and c-2 to c-10, may be selected from the group consisting of the above b-7 to b-9 and c-7 to c-9, or may be the structure represented by b-8.

The above-mentioned arylene group, aryleneoxy group, and arylenethio group, which are examples of X⁰, may be independently substituted with a group selected from the group consisting of a halogen atom (e.g., a fluorine atom, a chlorine atom, a bromine atom, or an iodine atom, preferably a fluorine atom or a chlorine atom, and more preferably a fluorine atom), a methyl group, and a trifluoromethyl group. The number of substituents may be more than one. In this case, the substituents may be the same or different from one another. The substituent suitable for the arylene group, the aryleneoxy group, and the arylenethio group is a fluorine atom and/or a trifluoromethyl group, particularly a fluorine atom. In the case where Y contains no fluorine atom, the arylene group, the aryleneoxy group, and the arylenethio group are preferably substituted with at least one or more fluorine atoms.

In the above formula (I), the divalent organic group represented by Y is not particularly limited and may be, for example, a divalent organic group having an aromatic ring. More specifically, the divalent organic group having an aromatic ring may be a group having one benzene ring; or a group having a structure in which two or more benzene rings are coupled directly or via a carbon atom (namely, a single bond or an alkylene group), an oxygen atom, or a sulfur atom. Specific examples include the following groups.

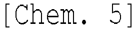

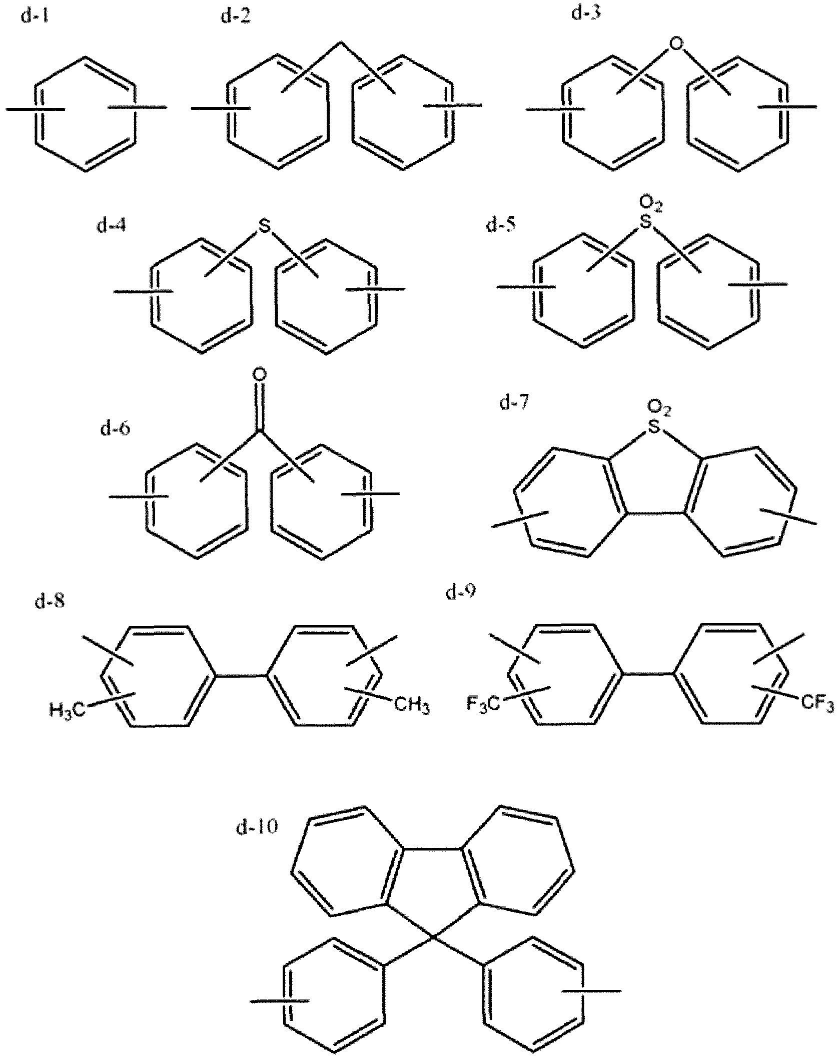

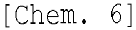

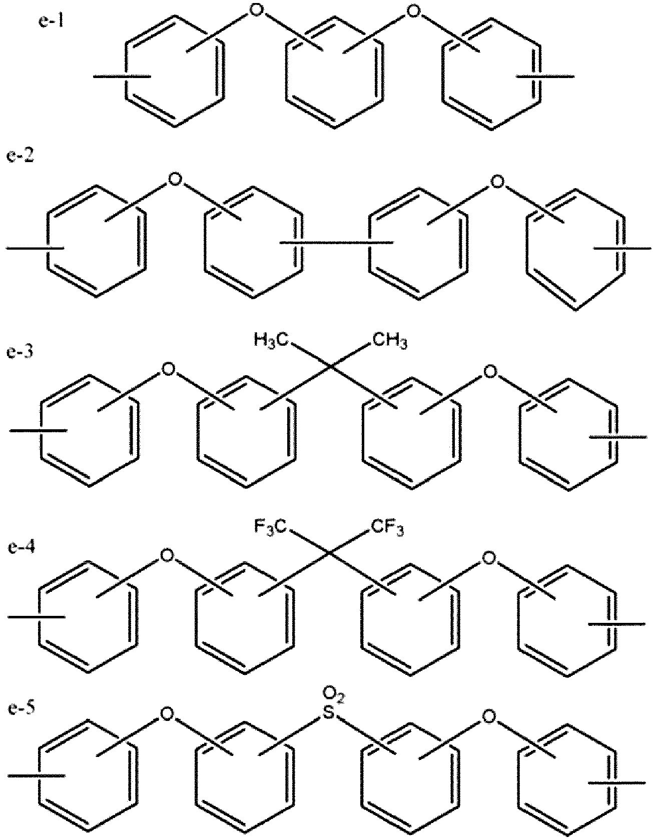

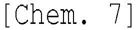

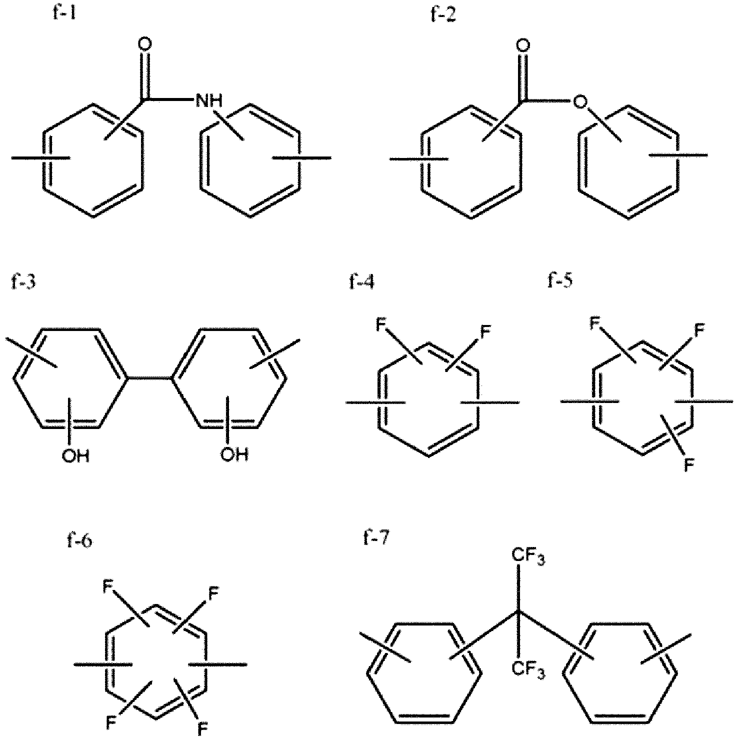

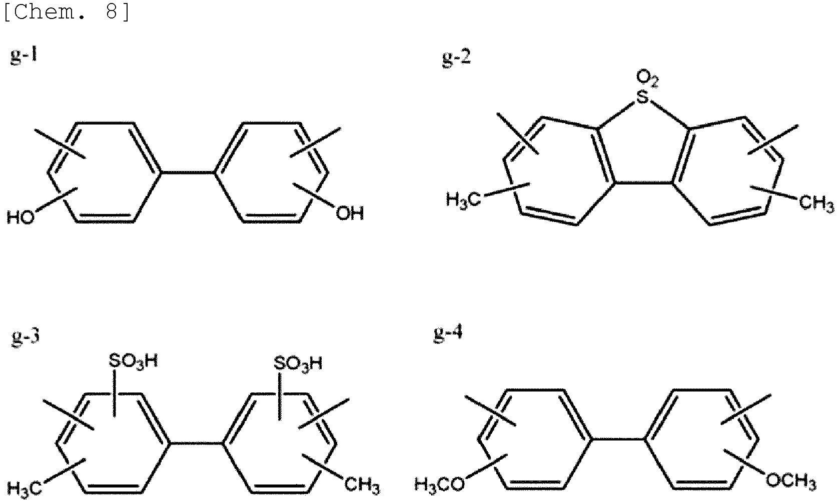

When possible, the above-mentioned divalent organic group having an aromatic ring, which is an example of Y, may be substituted with a group selected from the group consisting of a halogen atom (e.g., a fluorine atom, a chlorine atom, a bromine atom, or an iodine atom, preferably a fluorine atom or a chlorine atom, and more preferably a fluorine atom), a methyl group, and a trifluoromethyl group. The number of substituents may be more than one. In this case, the substituents may be the same or different from one another. Particularly in the case where X⁰ contains no fluorine atom, the substituent suitable for the divalent organic group having an aromatic ring is preferably a fluorine atom and/or a trifluoromethyl group, and more preferably a fluorine atom.

In view of spheroid-forming efficiency, Y in the above formula (I) is preferably a structure selected from the group consisting of d-3, d-9, e-1 to e-4, f-6, and f-7, and more preferably a structure of e-1, e-3, or e-4.

In the above formula (I), Z¹, Z², Z³, Z⁴, Z⁵, and Z⁶ may be the same or different from one another and are independently selected from a hydrogen atom, a fluorine atom, a chlorine atom, a bromine atom, and an iodine atom. In the case where at least one of X⁰ and Y contains no fluorine atom, at least one of Z¹, Z², Z³, Z⁴, Z⁵, and Z⁶ is preferably a fluorine atom.

In view of spheroid-forming efficiency, the divalent organic group represented by X⁰ in the above formula (I) is selected from the group consisting of -C(CF₃)₂-, the above b-2 to b-10, and c-2 to c-10; and Y is selected from the group consisting of d-3, d-9, e-1 to e-4, f-6, and f-7 in one preferable embodiment of the present invention. In a more preferable embodiment of the present invention, the divalent organic group represented by X⁰ in the above formula (I) is selected from the group consisting of -C(CF₃)₂-, b-7 to b-9, and c-7 to c-9; and Y is selected from the group consisting of e-1, e-3, and e-4.

The polyimide resin having a structural unit represented by formula (I) can be obtained by calcination of a polyamic acid obtained by polymerization of an acid dianhydride and a diamine . The imidization degree of the "polyimide resin having a structural unit represented by formula (I)" does not have to be 100%. That is, the polyimide resin having a structural unit represented by formula (I) may have only a structural unit represented by the above formula (I), or alternatively may partially have a structural unit in which an amic acid remains as is and is not converted to a cyclic imide structure due to insufficient cyclodehydration, as long as the desired effects of the present invention are not impaired.

The synthesis reaction of the polyamic acid is preferably performed in an organic solvent. The organic solvent used for the synthesis reaction of the polyamic acid is not particularly limited as long as the organic solvent allows a reaction of an acid dianhydride and a diamine to proceed efficiently and is inert to these compounds. Examples of the organic solvent include polar solvents, such as N-methyl pyrrolidone (NMP), N,N-dimethylacetamide, N,N-dimethylformamide, tetrahydrofuran, dimethyl sulfoxide, sulfolane, methyl isobutyl ketone, acetonitrile, benzonitrile, nitrobenzene, nitromethane, acetone, methyl ethyl ketone, isobutyl ketone, and methanol; nonpolar solvents, such as xylene and toluene; etc. Among these examples, polar solvents are preferred. One of these organic solvents may be used alone, and also a mixture of two or more of them may be used. The reaction mixture after amidation may be directly subjected to thermal imidization. The concentration of the polyamic acid in the solution of the polyamic acid is not particularly limited and is preferably 5 wt% or more, more preferably 10 wt% or more; and is preferably 50 wt% or less, more preferably 40 wt% or less in view of the polymerization reactivity of the resin composition, its viscosity after polymerization, and its ease of handling in subsequent film production and calcination. The viscosity of the resin composition is not particularly limited and can be measured according to any known measurement method. The viscosity of the resin composition is, for example, in the range of 1 to 20 Pa·s, preferably 3 to 15 Pa·s as measured at 23°C.

The polyamic acid is subjected to thermal or chemical imidization to yield a resin composition comprising a fluorine-containing polyimide. In a specific embodiment, the polyamic acid is subjected to heat treatment for imidization (thermal imidization) to yield a resin composition comprising a fluorine-containing polyimide. The polyimide obtained by thermal imidization is preferable for cell culture applications because the reaction product does not contain any residual catalyst.

In the case of thermal imidization, for example, the polyamic acid is calcinated under an air atmosphere, preferably an inert gas atmosphere, such as a nitrogen, helium, or argon atmosphere, or under vacuum, preferably at a temperature of 50 to 400°C, more preferably 100 to 380°C, preferably for 0.1 to 10 hours, more preferably 0.2 to 5 hours, to yield a resin composition comprising a polyimide.

The polyamic acid used for thermal imidization is preferably in the form of a solution in a suitable solvent. The solvent may be any solvent that can dissolve the polyamic acid. The solvent may be any of the solvents listed above for the synthesis reaction of the polyamic acid.

In the case of chemical imidization, the polyamic acid can be directly converted to an imide by a reaction using the cyclodehydration reagent described below in a suitable solvent.

The cyclodehydration reagent is not particularly limited as long as it is capable of chemically cyclodehydrating the polyamic acid into a polyimide. For efficient imidization, a tertiary amine compound alone or a combination of a tertiary amine compound and a carboxylic anhydride is preferably used as the cyclodehydration reagent.

Examples of the tertiary amine compound include
trimethylamine, triethylamine, tripropylamine, tributylamine,
pyridine, 1,4-diazabicyclo[2.2.2]octane (DABCO),
1,8-diazabicyclo[5.4.0]undec-7-ene,
1,5-diazabicyclo[4.3.0]non-5-ene,
N,N,N',N'-tetramethyldiaminomethane,
N,N,N',N'-tetramethylethylenediamine,
N,N,N',N'-tetramethyl-1,3-propanediamine,
N,N,N',N'-tetramethyl-1,4-phenylenediamine,
N,N,N',N'-tetramethyl-1,6-hexanediamine,
N,N,N',N'-tetraethylmethylenediamine,
N,N,N',N'-tetraethylethylenediamine, etc. Among these
examples, pyridine, DABCO, and
N,N,N',N'-tetramethyldiaminomethane are preferred, and DABCO is more preferred. One kind of tertiary amine or two or more kinds of tertiary amines may be used.

Examples of the carboxylic anhydride include acetic anhydride, trifluoroacetic anhydride, propionic anhydride, butyric anhydride, isobutyric anhydride, succinic anhydride, maleic anhydride, etc. Among these examples, acetic anhydride and trifluoroacetic anhydride are preferred, and acetic anhydride is more preferred. One kind of carboxylic anhydride or two or more kinds of carboxylic anhydrides may be used.

The solvent used for dissolution of the polyamic acid in chemical imidization is preferably a polar solvent that is highly capable of dissolving the polyamic acid. Examples of such a solvent include tetrahydrofuran, N,N-dimethylacetamide, N,N-dimethylformamide, N-methyl pyrrolidone, dimethyl sulfoxide, etc. Among these examples, one or more kinds selected from the group consisting of N,N-dimethylacetamide, N,N-dimethylformamide, and N-methyl pyrrolidone are preferred in order for the reaction to proceed uniformly. In the case where these solvents are used as the solvent for amidation, the reaction mixture after amidation can be directly used for chemical imidization as it is without isolation of the polyamic acid.

The weight-average molecular weight of the polyimide resin is, for example, 5,000 to 2,000,000, preferably 8,000 to 1,000,000, and more preferably 20,000 to 500,000. As used herein, the weight-average molecular weight of the resin can be measured according to any known measurement method. When the weight-average molecular weight is within the above range, favorable results can be obtained in terms of synthesis of the polyimide resin, its ease of handling, film formation, and spheroid-forming efficiency.

The cell-adhesive surface may further comprise an additive component such as a plasticizer or an antioxidant, in addition to the cell-adhesive substance.

The cell-adhesive substance or surface [the hydrophobic (particularly, not ultra-hydrophobic) cell-adhesive substance or surface] may have a static water contact angle of 70° or more and/or a sliding angle of 15° or more. It may have a static water contact angle of 70° or more and a sliding angle of 15° or more. When the cell-adhesive surface meets these requirements, spheroid formation thereon is further facilitated.

In view of spheroid-forming efficiency, the static water contact angle is preferably more than 75°, more preferably 77° or more, still more preferably 79° or more, and particularly preferably 80° or more (e.g., more than 80°). When the upper limit of the static water contact angle is specified, the static water contact angle is, for example, less than 150°, preferably 120° or less (e.g., less than 120°), more preferably 110° or less, still more preferably 100° or less (e.g., less than 99°, 98° or less, 97° or less, 95° or less, etc.), and particularly preferably less than 90°.

On the other hand, the cell-adhesive substance or surface [the hydrophilic (particularly, not ultra-hydrophilic) cell-adhesive substance or surface] may have a static water contact angle of preferably 65° or less, more preferably 55° or less, and still more preferably 50° or less. When the lower limit is specified, the static water contact angle may be 0° or more, preferably 5° or more, and more preferably 10° or more.

In view of spheroid-forming efficiency, the sliding angle of the cell-adhesive substance or surface may be 18° or more, 19° or more, 20° or more, 22° or more, 24° or more, 26° or more, 28° or more, or 30° or more, and among these examples, a higher sliding angle is more preferable. When the upper limit of the sliding angle is specified, the sliding angle is, for example, less than 80°, preferably 70° or less (e.g., less than 70°), more preferably 60° or less (e.g., less than 60°), and still more preferably 50° or less (e.g., less than 50°). The aforementioned surface properties can be measured according to known methods.

The cell-adhesive surface of the cell culture sheet may be the whole or part of the sheet surface, partitioned, or flat as long as the surface to be in contact with cells is a cell-adhesive surface. For example, the entire surface of the cell culture sheet may be a flat cell-adhesive surface. When the cell culture sheet has protrusions and recesses on its surface and the cell culture surface is partitioned, the part on which the cells adhere is made of a cell-adhesive surface.

When the surface of the cell culture sheet is a flat cell-adhesive surface, it may have some unevenness as long as the seeded cells can settle and adhere on it, and regardless of the unevenness, the entire surface may be where the cells adhere.

The cell culture sheet, which is a flat cell-adhesive surface, may be formed of a cell-adhesive substance physically or chemically immobilized or disposed on the surface of the substrate. Alternatively, the sheet itself may be formed of a cell-adhesive substance.

For immobilization of the substance on the substrate surface, various method can be used. For example, a solution containing the substance may be applied and dried on the substrate surface; the substance may be melted and compression-bonded to the substrate surface; the substance may be applied on the substrate surface and cured with energy ray such as UV; the functional group of the substance may be covalently bonded to the functional group of the substrate by chemical reaction (e.g., condensation between certain functional groups, such as a carboxyl group and an amino group); or the thiol group of the substance may be bonded to a thin film of metal (platinum, gold, etc.) formed on the substrate in advance. The thickness of a layer of the substance immobilized on the substrate surface is not particularly limited and is, for example, 0.01 to 1,000 µm. For sheet formation, for example, on a sheet having a release coated surface (e.g., an organic polymer film such as a polyethylene substrate, ceramic, metal, glass, etc.), the substance is applied at an appropriate thickness by casting, spray coating, dip coating, spin coating, roll coating, or other techniques, and then heated to form a sheet-like layer of the substance.

When the cell culture sheet has protrusions and recesses on its surface, it is preferable that cells adhere on the base of the recess. In one embodiment, the base is composed of the cell-adhesive substance. The inner circumferential face of the recess is not particularly limited and may, for example, be composed of a non-cell adhesive substance. The cell culture sheet may be a cell culture sheet having a micropattern formed of a cell-adhesive surface and a non-cell adhesive surface. Such a cell culture sheet is, for example, a sheet having a cell-adhesive substance layer and a micropattern of a non-cell adhesive substance on the surface of the cell-adhesive substance layer (i.e., a sheet having a micropatterned mask of a non-cell adhesive substance on a cell-adhesive substance layer).

It is assumed that cell culture sheets with shallow recesses, such as cell culture sheets having recesses formed in a micropattern, contribute to the expression of cell functions better than cell culture vessels in which cells are cultured in individual wells or cavities separated by high side walls, such as conventional microtiter plates or microplates. This is because, after seeding on such a cell culture sheet, cells are placed on the cell-adhesive surface of individual shallow recesses (compartments), but the medium over the recesses covers the entire sheet, and humoral factors are shared by all the seeded cells, allowing cell-cell interaction and thus improved expression of cell functions.

The formation of the micropattern can be performed by, for example, microcontact printing, spin coating, casting, roll coating, die coating, gravure coating, spray coating, bar coating, flexographic printing, dip coating, inkjetting, or a patterning method in which a desired substance is injected into the spaces of a protruded and recessed surface of a base material by capillary force generated in the spaces.

The non-cell adhesive substance is not particularly limited as long as the substance does not bind to cell surface molecules, such as proteins and sugar chains, present on the cell membrane of the cells used for culture. The non-cell adhesive substance may or may not be biocompatible. The non-cell adhesive substance may be hydrophobic or hydrophilic. The non-cell adhesive substance may be, for example, ultra-water repellent (ultra-hydrophobic) or ultra-hydrophilic. For cell adhesion prevention, homogeneous spheroid production, efficient spheroid formation, etc., hydrophobic (particularly, ultra-hydrophobic) [e.g., more hydrophobic (particularly, ultra-hydrophobic) as compared with a hydrophobic or hydrophilic (particularly, hydrophobic) cell-adhesive surface or a resin which constitutes such a cell-adhesive surface (further the contact angle thereof)] substances are particularly preferred. In addition, hydrophilic (particularly, ultra-hydrophilic) [e.g., more hydrophilic (particularly, ultra-hydrophilic) as compared with a hydrophilic or hydrophobic (e.g., hydrophobic) cell-adhesive surface or a resin which constitutes such a cell-adhesive surface (further the contact angle thereof)] substances are also preferred.

Examples of such a substance include polyethylene glycol and its derivatives, compounds including 2-methacryloyloxyethyl phosphorylcholine (MPC) and its derivatives and hydroxyethyl methacrylate (HEMA) and its derivatives or polymers thereof, and compounds including segmented polyurethane (SPC) and its derivatives; proteins (albumin etc.) harvested from the living body; and non-cell adhesive sugar chains (agarose, cellulose, etc.). From these substances, an appropriate one can be selected according to the kind of the cells. In particular, MPC and its derivatives or polymers thereof are preferred because these compounds have favorable adhesion to synthetic resins and contribute to simplifying the production process of the cell culture sheet and to more homogeneous production of spheroids.

The non-cell adhesive substance may be modified as appropriate for ease of handling, the desired level of hydrophobicity (e.g., ultra-hydrophobicity) or hydrophilicity (e.g., ultra-hydrophilicity), etc. For example, a hydrophilic substance may be subjected to crosslinking etc. so that it has both hydrophilicity and low water solubility. In another example, a raw material (e.g., hydrophobic or hydrophilic material) may be hydrophobized or hydrophilized (e.g., subjected to introduction of a hydrophobic or hydrophilic group etc.) to yield a material having the desired hydrophobicity or hydrophilicity.

The non-cell adhesive surface is defined, for example, as the surface described as follows. When cells in a solution used for culture settle on the surface, the cells almost completely maintain their shapes and do not adhere on the surface at all or even if they temporarily adhere on the surface weakly, they spontaneously come off afterwards. The non-cell adhesive surface is, for example, formed of a non-cell adhesive substance immobilized physically or chemically on a sheet surface having recesses. Alternatively, the sheet itself may be formed of a non-cell adhesive substance.

In order to estimate whether the non-cell adhesive surface is effective to enhance the uniformity in size and circularity of spheroids, surface properties such as static water contact angle (described later) can be used as an indicator.

For example, in the case where the non-cell adhesive surface is a hydrophobic surface formed of the hydrophobic substance described above, the static water contact angle is preferably 90° or more, more preferably 93° or more, and still more preferably 95° or more. The static water contact angle may be 150° or less and is preferably 130° or less, more preferably 120° or less.

On the other hand, in the case where the non-cell adhesive surface is a hydrophilic surface, the static water contact angle is preferably 65° or less, more preferably 55° or less, and still more preferably 50° or less. The static water contact angle may be 0° or more and is preferably 5° or more, more preferably 10° or more.

For example, when a highly hydrophobic MPC (or a surface formed of such an MPC) is used, the static water contact angle will be, for example, 90° or more or 100° or more.

The static water contact angle may be a static water contact angle measured using a non-cell adhesive surface or a static water contact angle measured using a non-cell adhesive substance (or a substance constituting a non-cell adhesive surface).

The static water contact angle may be measured by, for example, the method described later etc.

In order to enhance the uniformity in size and circularity of spheroids, it is preferable to optimize the balance between the cell adhesiveness of the cell-adhesive surface and that of the non-cell adhesive surface. In this context, the non-cell adhesive surface may have some level of cell adhesiveness as long as its level is lower than that of the cell-adhesive surface. For example, in the case where the static water contact angle is used as an indicator, the difference in static water contact angle (or an absolute value thereof) between the cell-adhesive surface (or the cell-adhesive substance) and the non-cell adhesive surface (or the non-cell adhesive substance) is preferably 3° or more (e.g., 5° or more), more preferably 10° or more (e.g., 12° or more), and still more preferably 15° or more. In addition, the upper limit of the difference in static water contact angle (or an absolute value thereof) can be determined as appropriate for the combination of hydrophobicity and hydrophilicity of the cell-adhesive surface (or the cell-adhesive substance) and the non-cell adhesive surface (or the non-cell adhesive substance), etc. and is not particularly limited. For example, the upper limit may be 100°, 90°, 80°, 70°, 60°, 50°, 40°, 30°, etc.

A specific example of the cell culture sheet having protrusions and recesses on its surface is a cell culture sheet comprising a plurality of recesses each having an opening of 1, 000 µm or less in diameter, wherein each recess has an inner circumferential face and a bottom face, wherein the inner circumferential face has a non-cell adhesive surface, and wherein the bottom face has a cell-adhesive surface.

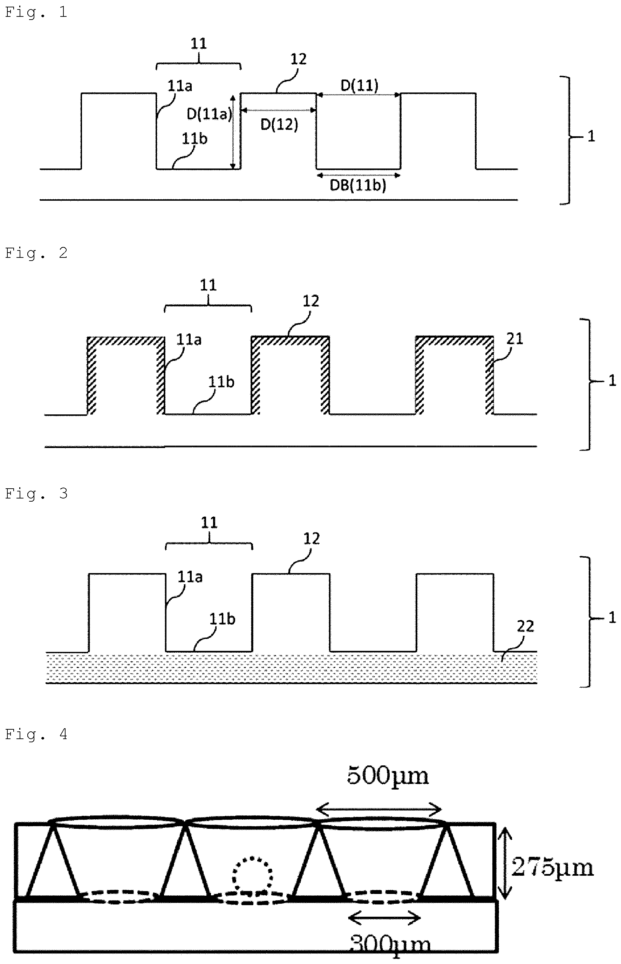

Here, the structure of the cell culture sheet having protrusions and recesses on its surface will be described using a vertical cross-sectional view through recesses of the sheet. As shown in the schematic view of Fig. 1, a plurality of recesses 11 are present at a sheet surface 12, and each recess 11 has an inner circumferential face 11a and a bottom face 11b. The recess 11 is a space used for spheroid formation.

The number of recesses 11 at the sheet surface 12 depends on the area (dimension) of the sheet and the type of the cells to be cultured and cannot be definitely determined, but an appropriate number for each individual case can be selected according to the common technical knowledge. For example, the minimum number per unit area (cm²) may be 1, 10, 30, 50, etc. The maximum number per unit area (cm²) may be 1,000, 500, 300, 200, 100, etc. The total number of recesses at the sheet surface can be determined as appropriate and is, for example, 10 or more, 100 or more, 1,000 or more, 10,000 or more, 50,000 or more, etc.

The shape of the opening of the recess is not limited to a circle and may be, for example, a polygon or an ellipse. The size of the opening is, for example, 2,000 µm or less in diameter and can be determined as appropriate for the size of the cell to be cultured and the size of the desired spheroid according to the common technical knowledge. In the present invention, the size of the opening is a diameter (maximum length) of a bounding circle enclosing the opening regardless of the shape of the opening. The size of the opening is, for example, the length represented by D (11) in Fig. 1. The size of the opening is, for example, within the range of 10 to 2,000 µm, 10 to 1,000 µm, 10 to 800 µm, or 10 to 500 µm in diameter.

The shape of the bottom face of the recess is not limited to a circle and may be, for example, a polygon or an ellipse. In addition, the shape of the bottom face of the recess may be the same as or different from the shape of the opening. The bottom face may be flat (flat-bottomed shape) or curved. The area of the bottom face can be, for example, 1.0 × 10⁻¹ cm², 5.0 × 10⁻² cm², 2.0 × 10⁻² cm², 1.0 × 10⁻² cm², 5.0 × 10⁻³ cm², 2.5 × 10⁻³ cm², 2.0 × 10⁻³ cm², 1.0 × 10⁻³ cm², etc. per recess. The size (length) of the bottom face may be the same as or different from the size of the opening. The size of the bottom face may be smaller than the size of the opening or greater than the size of the opening. The size of the bottom face is, for example, the length represented by DB(11b) in Fig. 1. The size of the bottom face is, for example, within the range of 10 to 2,000 µm, 10 to 1,000 µm, 10 to 800 µm, 10 to 500 µm, 10 to 400 µm, or 10 to 300 µm in diameter. In the case where the size of the bottom face is equal to the size of the opening, the recess is formed in the shape of a cylinder. In the case where the size of the bottom face is smaller than the size of the opening, the recess is formed in a shape tapered to the bottom face.

The ratio of the size of the bottom face to the size of the opening (the size of the bottom face/the size of the opening) is not particularly limited and is, for example, 5/1 to 1/5, 3/1 to 1/3, or 1/1 to 1/2 in view of ease of cell seeding and collection.

The distance (gap) between adjacent openings is, for example, the length represented by D(12) in Fig. 1. The distance is not particularly limited and can be determined as appropriate for the desired mass culture. For example, the distance is a finite value in the range of 800 µm or less, 700 µm or less, 600 µm or less, 500 µm or less, 300 µm or less, 200 µm or less, 100 µm or less, etc.

The depth of the recess can be determined as appropriate for the size of the cell to be cultured according to the common technical knowledge. The depth of the recess is, for example, the length represented by D(11a) in Fig. 1. For example, the depth of the recess is within the range of 10 to 1,500 µm, 10 to 1, 000 µm, 10 to 800 µm, 10 to 500 µm, or 10 to 300 µm. When the recesses are formed in a micropattern, the depth of the recess can be less than 10 µm. In this case, the lower limit of the depth of the recess is a minimum thickness that allows us to discern non-attachment and attachment of cells. For example, the depth can be 100 nm to 500 nm.

The ratio of the size of the opening to the depth of the recess (the size of the opening/the depth of the recess) is not particularly limited and is, for example, 5/1 to 1/5, 3/1 to 1/3, or 2/1 to 1/1 in view of ease of cell seeding and collection. When the ratio is within this range, cells are unlikely to accidentally jump out of the recess, and operations such as cell collection and degassing can easily be done.

The thickness of the bottom face of the recess is not particularly limited, and an appropriate thickness can be selected according to the common technical knowledge.

The inner circumferential face of the recess has a non-cell adhesive surface, and the bottom face of the recess has a cell-adhesive surface. More specifically, for example, as shown in Figure 1, the inner circumferential face 11a has a non-cell adhesive surface, and the bottom face 11b has a cell-adhesive surface. In the cell culture sheet, the non-recessed face, i.e., the sheet surface, preferably has a non-cell adhesive surface in order to simplify the production of the cell culture sheet. The non-cell adhesive surface of the sheet surface may be the same as or different from the non-cell adhesive surface of the inner circumferential face of the recess. In the case where the non-cell adhesive surface of the sheet surface is the same as the non-cell adhesive surface of the inner circumferential face of the recess, the sheet surface and the inner circumferential faces of the recesses have a continuous surface.

The proportion of the cell-adhesive surface in the bottom face of the recess is not particularly limited and is preferably 90% or more, 95% or more, 99% or more, or substantially 100% of the area of the bottom face of the recess.

The proportion of the non-cell adhesive surface in the inner circumferential face of the recess is not particularly limited and is preferably at a level that would result in less adhesion of cultured cells. For example, the proportion is preferably 90% or more, more preferably 95% or more, and still more preferably 100% of the area of the inner circumferential face. The proportion of the non-cell adhesive surface in the sheet surface (the non-recessed face) is not particularly limited and is preferably 90% or more, 95% or more, 99% or more, or substantially 100% of the area of the non-recessed face. The proportion of the non-cell adhesive surface in the combined total area of the non-recessed face and the inner circumferential faces of the recesses is preferably 90% or more, 95% or more, 99% or more, or substantially 100% of the combined total area of the non-recessed face and the inner circumferential faces of the recesses.

For example, as shown in the schematic view of Fig. 2, the non-cell adhesive substance 21 is immobilized at the sheet surface 12 and the inner circumferential face 11a of the recess 11. The sheet formation from or immobilization of the non-cell adhesive substance can be performed according to the procedure described above for the cell culture sheet which is a flat cell-adhesive surface.

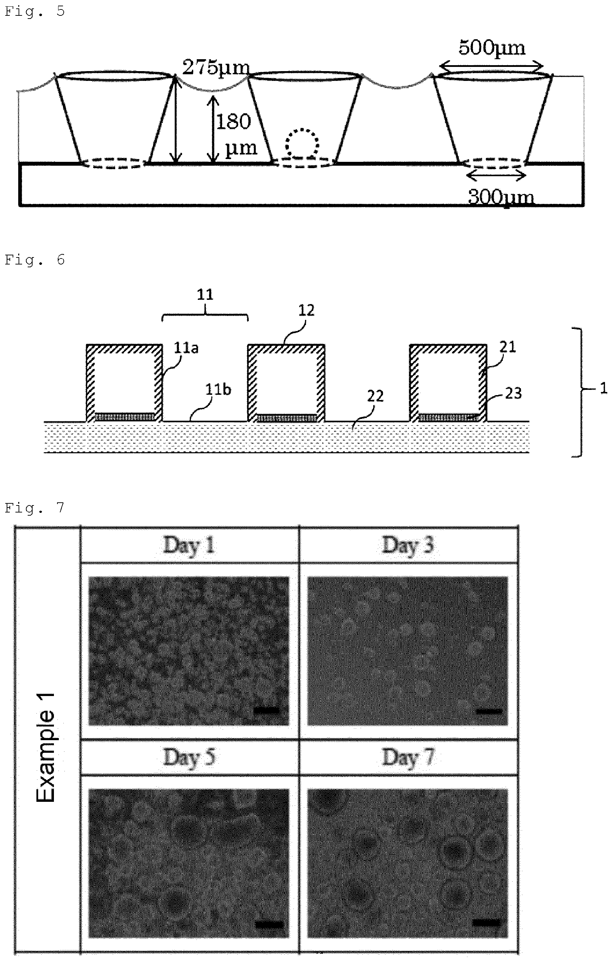

The cell culture sheet having protrusions and recesses on its surface, which comprises a plurality of recesses configured as described above, may be a layered structure comprising a layer including the bottom faces of the recesses and a layer including the inner circumferential faces of the recesses. The layer including the inner circumferential faces of the recesses is a layer having through-holes. Therefore, in one embodiment, the cell culture sheet is a laminate comprising a layer having the non-cell adhesive surface and a layer having the cell-adhesive surface, wherein the layer having the non-cell adhesive surface has through-holes. For example, as shown in the schematic view of Fig. 3, the bottom face 11b of the recess 11 may be a part of a layer of a cell-adhesive substance 22, and on top of the layer, another layer having a non-cell adhesive surface with through holes may be formed. For reference, schematic views of a spheroid formed in a recess of the cell culture sheet are shown in Figs. 4 and 5.

In order to facilitate through-hole formation and simplify the production of the cell culture sheet, the layer having a non-cell adhesive surface with through holes is preferably a layer of a non-cell adhesive substance immobilized on a layered substrate.

The layered substrate may be any layered substrate known in the technical field. Examples of the layered substrate include plates formed of synthetic resins, such as polystyrene, polyethylene, polypropylene, polycarbonate, polyamide, polyacetal, polyester (polyethylene terephthalate etc.), polyurethane, polysulfone, polyacrylate, polymethacrylate, polyvinyl, polycycloolefin, polyether ketone, polyether ether ketone, polyimide, and silicone; synthetic rubbers, such as EPDM (ethylene propylene diene monomer), and natural rubbers; glasses; ceramics; metallic materials, such as stainless steel; etc. In addition, a preferable embodiment of the substrate is a transparent substrate.

In view of workability, the immobilization of the non-cell adhesive substance on the layered substrate preferably follows the step of through-hole formation, which is described later.

The wall of the through-hole preferably corresponds to the inner circumferential face of the recess described above. The sizes and shapes of the opening and the opposite end of the through-hole can be determined in the same manner as described above for the recess . The depth of the through-hole corresponds to the thickness of the layer having the non-cell adhesive surface and also to the depth of the recess described above. The depth of the through-hole can be determined in the same manner as described above for the recess. In the case where the non-cell adhesive substance is immobilized on the layered substrate, the layer of the non-cell adhesive substance has a thickness of, for example, preferably 1 nm or more, more preferably 10 nm or more. As long as the thickness of the whole layer including the layer of the non-cell adhesive substance and the substrate is within the above-mentioned range of the thickness of the layer having the non-cell adhesive surface, an appropriate thickness of the layer of the non-cell adhesive substance can be selected.

The method for through-hole formation is not particularly limited and may be any method that enables through-holes of the above-specified size to be formed. For example, piercing (drills etc.), optical microprocessing (lasers (e.g., a CO₂ laser, an excimer laser, a semiconductor laser, a YAG laser) etc.), etching, embossing, and other processing techniques can be used for through-hole formation. Tapered through-holes may be formed by such processing techniques. In this case, the surrounding area of the end of the through-hole may be deformed, that is, the thickness of the layer at the periphery of the opening may be different from that at the midpoint between adjacent openings, for example, as shown in Fig. 5.

The thickness of the layer having the cell-adhesive surface is, for example, 1 to 4 mm, preferably 1 µm to 1 mm. The thickness of the layer having the cell-adhesive surface may be the same as that of the bottom face of the recess.

In one embodiment, the cell culture sheet further comprises an adhesive layer between the layer having the cell-adhesive surface and the layer having the non-cell adhesive surface. For example, in one embodiment shown in Fig. 6, the cell culture sheet comprises an adhesive layer 23 between a layer of a cell-adhesive substance 22 and a portion having a non-cell adhesive substance 21 superficially immobilized.

The adhesive layer may be any known adhesive layer in the technical field. Examples of the adhesive layer include adhesive layers made of acrylic resins, silicone-based resins, synthetic rubbers, natural rubbers, etc. Preferred are low-dissolution adhesive layers. Commercial double-sided tapes etc. also may be used.

The thickness of the adhesive layer is not particularly limited, and an appropriate thickness can be selected as long as the effects of the present invention are not impaired. For example, the thickness may be 0.5 to 100 µm.

The cell culture sheet can be produced by laminating a layer having a non-cell adhesive surface with through-holes and a layer having a cell-adhesive surface in this order. The cell culture sheet may further comprise a layer other than the layers described above. A cavity-containing layer may be comprised in the cell culture sheet.

For laminating the layers, the layers may be separately prepared in advance and laminated to one another, or alternatively, a layer may be formed on another layer prepared in advance. Alternatively, these methods may be employed in combination. More specifically, for example, on a sheet having a release coated surface (e.g., an organic polymer film such as a polyethylene substrate, ceramic, metal, glass, etc.), a cell-adhesive substance is applied at an appropriate thickness by casting, spray coating, dip coating, spin coating, roll coating, or other techniques, and then heated to form a sheet-like layer having a cell-adhesive surface. Separately, through-holes are formed in a substrate, and the surface is coated with a non-cell adhesive substance to prepare a layer having a non-cell adhesive surface. Subsequently, after removal of the release sheet of the layer having a cell-adhesive surface, the layer having a non-cell adhesive surface prepared separately is laminated to the layer having a cell-adhesive surface to produce the above-described laminate. For laminating the layer having a non-cell adhesive surface and the layer having a cell-adhesive surface, the adhesive layer described above may be used, or alternatively, welding (high frequency welding, ultrasonic welding, etc.) or compression bonding (thermocompression bonding etc.) may be employed.

The thickness of the cell culture sheet is not particularly limited and is preferably 10 to 5,000 µm, more preferably 10 to 2,000 µm in view of ease of handling. The area (dimension) of the cell culture sheet is not particularly limited and is, for example, 0.01 to 10,000 cm², preferably 0.03 to 5,000 cm².

In order that the thus obtained cell culture sheet can be directly placed in a known cell culture device, the cell culture sheet may be subjected to sizing as appropriate for the size of the device to be used, regardless of whether or not the cell culture sheet has protrusions and recesses. For cell culture, cell-containing medium is placed on the surface on one side of the cell culture sheet. More specifically, the cell culture sheet is placed and fixed in a cell culture vessel such as a culture plate, each well of a plate, a culture petri dish (culture dish), a flask, a culture bag, and other various types of cell culture vessels, and to the cell culture vessel, cell-containing medium is added to cover part or the entire surface of the fixed cell culture sheet. In this way, cell culture can be performed.

The undifferentiated cells to be cultured on the cell-adhesive surface of the cell culture sheet are not particularly limited as long as they have the ability to differentiate and are in an undifferentiated state. Specific examples of the cells that can be used include primary cells and established cell lines derived from organs and tissues (brain, liver, pancreas, spleen, heart, lung, intestine, cartilage, bone, fat tissue, kidney, nerve, skin, bone marrow, dental pulp, embryo, periosteum, synovium, muscle, placental tissue, umbilical cord tissue (umbilical cord blood), peripheral blood, etc.) of humans and non-human animals (monkeys, pigs, dogs, rabbits, rats, mice, etc.) and also include genetic engineered cells derived from the foregoing cells.

The integrin-expressing cells are not particularly limited as long as they are capable of expressing an integrin, and the integrin-expressing cells may be undifferentiated cells. Such cells can be the same as those described above.

More specific examples include stem cells and progenitor cells, such as embryonic stem cells (ES cells), induced pluripotent stem cells (iPS cells), neural stem cells, mesenchymal stem cells, tissue stem cell (somatic stem cells), hematopoietic stem cells, and cancer stem cells. One of these cells may be used alone, or alternatively two or more of them may be used in a mixture at any ratio.

The medium and culture conditions used in the culture of the undifferentiated cells or the integrin-expressing cells can be determined as appropriate for the cells to be used. In the present invention, since the cells are cultured on the cell-adhesive surface described above, the cells do not need to be cultured on feeder cells or on substrates coated with biological components such as collagen and can be cultured in the absence of feeder cells or components that have an effect on cell adhesion (e.g., collagen, hyaluronic acid, Matrigel, laminin, fibronectin, gelatin, etc.). The "feeder cell" refers to a cell that can provide a culture environment conditioned for undifferentiated cells or integrin-expressing cells to be cultured. For example, when the cells to be cultured are human ES cells, mouse fibroblasts can be used as feeder cells.

In the present invention, for example, undifferentiated cells or cells are seeded at a density of 5 × 10³ to 3 × 10⁴ cells/cm² on the cell-adhesive surface described above and cultured in serum-free medium (e.g., StemFit (registered trademark) medium) for 1 to 7 days to achieve the formation of undifferentiated cell spheroids, which are provided in one aspect of the present invention, or integrin-expressing cell spheroids, which are provided in another aspect of the present invention. If necessary, the cell culture sheet or cell culture vessel described above may be subjected to degassing before use. The degassing is not particularly limited, and examples include spraying, pipetting, shaking, thermal change such as heating and cooling, centrifugation, vacuum deaeration, ultrasonication, and other commonly used treatments. Preferred are spraying, pipetting, and thermal change. In addition, a medium containing a Rock inhibitor may be used at the time of cell seeding to prevent cell death due to dispersion during seeding. If necessary, cells may be expanded in advance and then treated with a detachment reagent before seeding. Examples of the detachment agent include trypsin, TrypLE^{™}, and Accutase^{™}.

The diameter of the thus obtained undifferentiated cell spheroid or integrin-expressing cell spheroid is not particularly limited and is, for example, 10 to 1,500 µm, 10 to 1,000 µm, 10 to 800 µm, 10 to 600 µm, or 10 to 500 µm. The diameter of the spheroid can be measured by the usual method (e.g., using an image-analysis software, a particle size analyzer, etc.) and can be expressed as, for example, a fluid diameter or an equivalent circle diameter. The shape of the spheroid can be spherical or dome-shaped (hemispherical) . In the case of a spherical shape, the circularity of the spheroid is, for example, 0.5 to 1.0, preferably 0.7 to 1.0.

The number of cells that form a single undifferentiated cell spheroid or a single integrin-expressing cell spheroid is not particularly limited, and a single spheroid may contain, for example, 1 × 10¹ cells or more, 1 × 10² cells or more, 1 × 10³ cells or more, 1 × 10⁴ cells or more, 1 × 10⁵ cells or more, 1 × 10⁶ cells or more, 1 × 10⁷ cells or more, 1 × 10⁸ cells or more, or 1 × 10⁹ cells or more. The upper limit of the number is not particularly specified, and the number of cells is, for example, 1 × 10¹⁰ cells or less.

In the present invention, since the spheroids are formed on the cell-adhesive surface described above, the risk of contamination is low, and for example, spheroids that do not contain components derived from serum can be formed although it depends on the constituent cells of the undifferentiated cell spheroid or the integrin-expressing cell spheroid. In general, cells are attached to each other and to the extracellular matrix in the body, but they are constantly in a suspended state in a vessel for suspension culture. Cells that cannot adhere may undergo apoptosis, and in order to prevent apoptosis, humoral factors contained in serum are required. However, the method of the present invention allows cells to adhere as in the body, thus eliminating the need for humoral factors to prevent apoptosis.

In addition to the spheroid formation on the cell-adhesive surface described above, another feature of the present invention is that the spheroids can be detached and collected without enzymatic treatment, so that the spheroids can be collected in an adherent state together with the extracellular matrix.

In another aspect of the present invention, spheroids express an integrin and thus have excellent cell adhesiveness and is capable of adhering well to wound sites.

The undifferentiated state of the undifferentiated cell spheroids can be confirmed by detecting the expression of an undifferentiation marker.

Examples of the undifferentiation marker include, but are not limited to, Oct3/4, Nanog, Sox2, POU5F1, c-Myc, and SSEA4. The undifferentiation marker can be detected and analyzed by any conventional method (e.g., real-time PCR, protein array, etc.). In one embodiment, the cell spheroid obtained by the method of the present invention has a relative mRNA expression level of the undifferentiation marker that is preferably at least 3-foldhigher, more preferably at least 5-foldhigher than the corresponding level in a cell spheroid obtained in suspension culture on a non-cell adhesive surface. The protein expression level of the undifferentiation marker is preferably at least 1.5-fold higher, more preferably at least 2-fold higher, and even more preferably at least 3-fold higher. The upper limits of these fold-change values are not particularly specified and are, for example, about 20-fold.

Integrins are proteins found on the surface of the cell membrane, are cell adhesion molecules, and are heterodimers consisting of two subunits, an α chain and a β chain. Examples of the integrin α chain include, but are not limited to, α1, α2, α3, α4, α5, α6, α7, α8, α9, α10, α11, αv, αIIb, etc. Examples of the integrin β chain include, but are not limited to, β1, β2, β3, β4, β5, β6, etc.

The integrin in the integrin-expressing cell spheroid is not limited as long as the protein level expression of at least one of the various subunits of the integrin α and β chains can be detected. The protein level expression can be analyzed by any conventional method (e.g., ELISA etc.). From the viewpoint of adhesion to wound sites, the integrin to be detected is preferably at least one of integrin αv, integrin β3, and integrin β6. In the case of human adipose-derived stem cells, from the viewpoint of the maintenance of their undifferentiated state, the integrin to be detected is preferably at least one of integrin αv, integrin α5, integrin α8, integrin αIIb, and integrin α11, and more preferably integrin αv. In one embodiment, the integrin-expressing cell spheroid obtained in another aspect of the present invention has a protein expression level of the integrin that is, for example, at least 1.2-fold higher, preferably at least 1.5-fold higher, more preferably at least 2-fold higher, and even more preferably at least 3-fold higher than the corresponding level in a cell spheroid obtained in suspension culture on a non-cell adhesive surface. The upper limit of this fold-change value is not particularly specified and is, for example, about 10-fold.

The protein expression level of the integrin in the integrin-expressing cell spheroid as determined per 1 × 10⁵ cells is not particularly limited, and when the integrin to be detected is integrin αv, the protein expression level may be, for example, 65 pg or more, and is preferably 70 pg or more, more preferably 75 pg or more. The upper limit of the protein expression level is not particularly specified and is, for example, 200 pg.

In one aspect of the present invention, undifferentiated cell spheroids can be produced, and the present inventors also found that the undifferentiated state of the resulting spheroids can be maintained by continuously culturing them on the cell-adhesive surface. Thus, the present invention also provides a method for maintaining cell spheroids in an undifferentiated state, the method comprising the step of culturing undifferentiated cell spheroids on a cell-adhesive surface of a cell culture sheet. In another aspect of the present invention, integrin-expressing cell spheroids can be produced. The cell culture sheet, medium, and culture conditions used in the method for maintaining cell spheroids in an undifferentiated state and the method for producing integrin-expressing cell spheroids can be specified in the same manner as in the method of the present invention for producing undifferentiated cell spheroids.

The resulting undifferentiated cell spheroid contain a large number of stable cells that maintain their undifferentiated state and thus can be used in research on various tissue/organ development and regeneration. In addition, the resulting undifferentiated cell spheroid can be induced to differentiate into various types of cells and thus can be used in regenerative medicine. In general, for inducing differentiation of undifferentiated cells, cell culture vessels coated with gelatin or the like are needed, but in one aspect of the present invention, induced differentiation can be efficiently achieved by continuously culturing the resulting undifferentiated cell spheroids on the cell-adhesive surface simply with the addition of a differentiation inducer, a differentiation promoter, etc.

In addition, since the resulting integrin-expressing cell spheroid has high integrin expression and thus has excellent cell adhesiveness, the cell spheroid is capable of adhering well to wound sites and is unlikely to undergo cell death. In particular, in the case of using undifferentiated cells, the resulting cell spheroid maintains its undifferentiated state well and thus has a high potential to provide therapeutic effects in regenerative medicine.

### EXAMPLES

Hereinafter, the present invention will be described in detail by examples, but the present invention is not limited thereto. In the following Examples, "room temperature" refers to a temperature of 20 to 30°C.

### Production Example 1

### Preparation of layer having a cell-adhesive surface (preparation of fluorine-containing polyimide film)

2.976 g (10.2 mmol) of 1,4-bis(aminophenoxy)benzene, 4.524 g (10.2 mmol) of 4,4'-(hexafluoroisopropylidene)diphthalic anhydride, and 42.5 g of N-methyl pyrrolidone were fed into a 100-mL three-necked flask. The mixture was stirred under a nitrogen atmosphere at room temperature and then left to stand for 5 days. Thus, a fluorine-containing polyamic acid resin composition (solid content: 15.0% by mass, 6FDA/TPEQ polyamic acid) was obtained. The weight-average molecular weight of the polyamic acid resin composition was 180, 000, and the viscosity was 14 Pa·s. The weight-average molecular weight of a polyamic acid and the weight-average molecular weight of a fluorine-containing polyimide after calcination are substantially the same.

The fluorine-containing polyamic acid resin composition obtained above was applied on a glass substrate using a die coater such that the thickness of the fluorine-containing polyimide film after calcination would be 40 µm. Subsequently, the coating was calcinated under a nitrogen atmosphere at 360°C for 1 hour. The calcined coating was separated from the glass substrate. Thus, a fluorine-containing polyimide film was obtained. The static water contact angle of the fluorine-containing polyimide film was 80.9°, and the sliding angle was 23.4°.

The methods for measuring the physical properties mentioned above are as follows.

### Measurement of weight-average molecular weight

Apparatus: HCL-8220GPC; manufactured by TOSOH
Column: TSKgel Super AWM-H
Eluent (LiBr·H₂O, NMP with phosphoric acid): 0.01 mol/L
Measuring method: A 0.5 wt% solution is prepared using an eluent, and the molecular weight is calculated based on a standard curve of polystyrene prepared in advance.

### Measurement of viscosity

Apparatus: VISCOMETER TV-22; manufactured by AS ONE Corporation
Setting: VI RANGE: H ROTOR No. 6 SPEED: 10 rpm
Standard liquids for calibrating viscometers: Nippon Grease JS14000
Measuring method: The viscosity is measured using 0.3 g of varnish after calibration with standard liquids for calibrating viscometers (measurement temperature: 23°C).

### Measurement of static water contact angle

Apparatus: Automatic contact angle meter (DM-500; manufactured by Kyowa Interface Science Co., Ltd.)
Measuring method: Two microliters of water is dropped onto a surface (a non-cell adhesive surface or a cell-adhesive surface) or a film (a film formed of a non-cell adhesive substance or a cell-adhesive substance), and then immediately the contact angle of the sessile water drop is measured (measurement temperature: 25°C).

### Measurement of sliding angle

Apparatus: Automatic contact angle meter (DM-500; manufactured by Kyowa Interface Science Co., Ltd.)
Measuring method: Twenty-five microliters of water is dropped onto a surface (a non-cell adhesive surface or a cell-adhesive surface) or a film (a film formed of a non-cell adhesive substance or a cell-adhesive substance), and the sheet is gradually tilted. The tilt angle at which the water drop begins to slide down is recorded as a sliding angle (measurement temperature: 25°C).

### Preparation of cell culture vessel

The obtained fluorine-containing polyimide film was placed on the bottom face of each well of a 24-well culture plate. Thus, a vessel used for cell culture was finally made.

### Example 1

The cells used were human iPS cells. The iPS cells were 201B7 cells, which were provided from Kyoto University.

### Expansion culture of cells

The cells established in the feeder cell culture system were acclimated to a feeder-free culture system using StemFit (registered trademark) AK02N (Ajinomoto) and iMatrix-511 (registered trademark) (Nippi). Cell detachment was performed in an aqueous solution of a 1:1 mixture of PBS containing 0.5 mM EDTA and TrypLE Select CTS^{™} (Thermo Fisher Scientific).

### Production of embryoid body (EB)

The iPS cells collected with the detachment reagent described above were suspended in StemFit (registered trademark) AK02N medium and seeded at 5.4 × 10⁴ cells/500 µL/well (2.7 × 10⁴ cells/cm²) in the cell culture vessel prepared in Production Example 1. At the time of seeding, the medium containing 10 µL of Y-27632 was used. One day after seeding, half of the medium was replaced, and after 4 days, the whole medium was replaced.

### Comparative Example 1

EBs were produced in the same manner as in Example 1, except that a PrimeSurface (registered trademark) 96U plate (manufactured by Sumitomo Bakelite) was used instead of the cell culture vessel used in Example 1. The seeding volume was 100 µL/well.

### Test Example 1 Microscopic observation

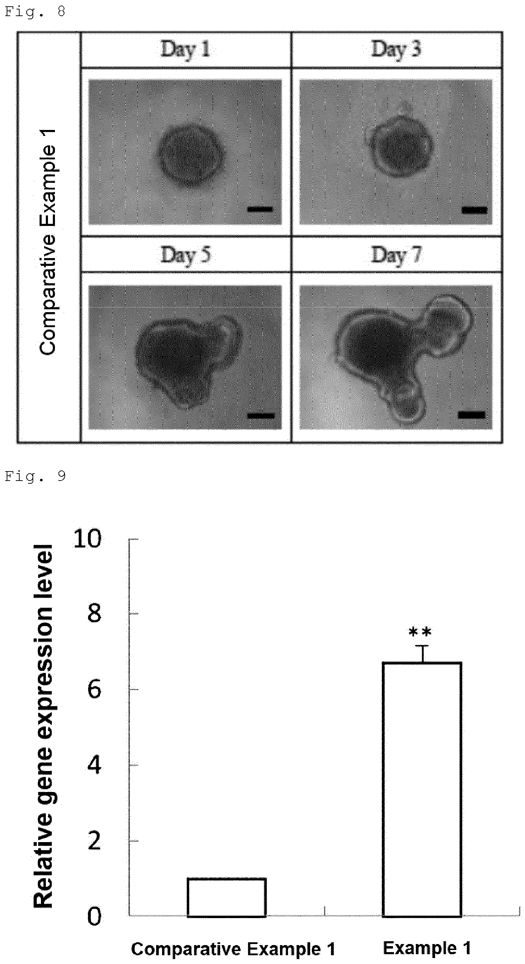

The morphology of EBs was microscopically observed on days 1, 3, 5, and 7 of culture. The results of Example 1 are shown in Fig. 7, and the results of Comparative Example 1 are shown in Fig. 8.

### Test Example 2 Evaluation of undifferentiated state of EBs

Seven days after seeding iPS cells, EBs were collected, and RNA was extracted using the RNeasy Mini Kit (Qiagen) . cDNA was synthesized from the RNA using ReverTra Ace (registered trademark) qPCR RT Master Mix (Toyobo) . The expression of the undifferentiation marker, POU5F1, was measured by real-time PCR using a pair of primers and a probe for POU5F1 in TaqMan^{™} Gene Expression Assay (FAM) (Thermo Fisher Scientific) (n = 3). The apparatus used for the measurement was StepOnePlus (Thermo Fisher Scientific) . GAPDH was used as the housekeeping gene. The obtained data were normalized to GAPDH, and the relative gene expression level was determined by the ΔΔCt method. The results are shown in Fig. 9.

As shown in the results, in Example 1, iPS cells began to form EBs on day 1 of culture, and a plurality of EBs were formed on day 3 of culture. On day 5 of culture, the size of EBs was increased to about 400 µm (Fig. 7). On the other hand, in Comparative Example 1, EBs (about 500 µm in size) were formed on day 1 of culture, but they did not adhere to the bottom of the wells. They sank at the bottom of the wells in a non-adherent state. The size was increased to about 600 µm on day 5, and the morphology was changed from a circular to an irregular shape (Fig. 8).

The level of POU5F1, an undifferentiation marker, in the EBs produced in Example 1 was significantly higher than that in the non-adherent EBs produced in Comparative Example 1 (Fig. 9). More specifically, the POU5F1 level in the EBs produced in Example 1 was 6.7-fold higher than that in the EBs produced in Comparative Example 1 (P < 0.01).

### Production Example 2

### Preparation of layer having a non-cell adhesive surface

A release tape on one side of a double-sided tape (25 µm in thickness) was removed, and a transparent PET film (250 µm in thickness) was laminated to the double-sided tape. On this laminate, through-holes of 300 µm in diameter were formed in a staggered configuration with a pitch of 500 µm using a CO₂ laser (formed through-holes: 400 holes/cm², 24000 holes/sheet, hole diameter on laser incidence side: 500 µm, hole diameter on a laser emission side: 300 µm). After that, on the surface of the PET film side, an MPC polymer solution (a 0.5% solution of a hydrophobic MPC polymer in ethanol) was applied using a spin coater (manufactured by MIKASA CO., LTD.: MS-A150) such that the final thickness would be 0.05 µm and dried in a dryer at 50°C for 2 hours. Thus, a layer having a non-cell adhesive surface (static water contact angle of the coating layer on the PET film (MPC polymer coating layer): 107.5°) was obtained.

### Preparation of cell culture sheet and culture vessel

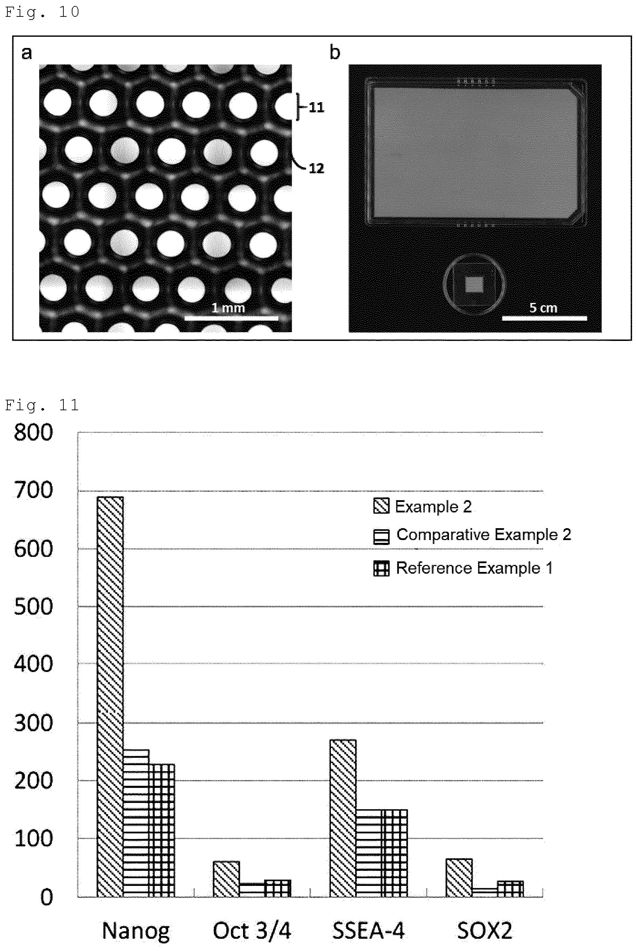

Next, the release tape on the opposite side of the double-sided tape from the non-cell adhesive surface was removed, and the layer having a cell-adhesive surface prepared as described in Production Example 1 was laminated to the double-sided tape to prepare a cell culture sheet (sheet thickness: 315 µm). A photograph of the prepared cell culture sheet taken from the bottom side of the recesses is shown in Fig. 10a. The obtained cell culture sheet was placed in a culture plate. Thus, a vessel used for cell culture was finally made. A photograph of the whole cell culture vessel taken from the bottom side of the recesses is shown in Fig. 10b.

### Example 2

The cells used were human adipose-derived stem cells (AdSCs) . The AdSCs used were a commercial product from Lonza (trade name: PT-5006) .

### Expansion culture of cells

Frozen cells were thawed in a constant temperature water bath at 37°C and added to 9 mL of KBM ADSC-2 medium (basal medium; manufactured by Kohjin-Bio Co., Ltd.) containing 5% FBS and 1% antibiotics. After centrifugation at 210 × g for 5 minutes, the supernatant was removed, and the cells were suspended in 1 mL of the basal medium. The cell suspension was added to two culture flasks (usable surface area: 225 cm²) at 1.0 × 10⁶ cells/30 mL medium/flask, and culture (expansion culture) was performed in a 5% (v/v) CO₂ incubator at 37°C.

### Degassing

The culture vessel made above was degassed. More specifically, about 1 mL of PBS was added to the vessel, pipetting was performed, and the vessel was placed in a 5% (v/v) CO₂ incubator at 37°C for 15 minutes. Then, after another pipetting operation, the PBS was removed with an aspirator, and 0.2 mL of KBM ADSC-2 medium containing 1% antibiotics was added to the vessel, which was then placed in a 5% (v/v) CO₂ incubator at 37°C overnight.

### Production of spheroids

The medium was removed from each culture flask, and 5 mL of a cell detachment solution, Accutase (manufactured by PromoCell), was added. The flasks were left to stand in a 5% (v/v) CO₂ incubator at 37°C for about 5 minutes for cell detachment. The cell detachment solution in each flask was recovered, and 10 mL of PBS was added to each flask for washing and transferred to a tube. After centrifugation at 210 × g for 5 minutes, the cells were suspended in 4 mL of KBM ADSC-2 medium containing 1% antibiotics, and the number of cells was counted. After that, the cell suspension was adjusted to 1.0 × 10⁶ cells/mL.

The medium in the culture vessel, which had been degassed in a 5% (v/v) CO₂ incubator at 37°C overnight, was removed, and the cells were seeded at 500 cells/recess in the culture vessel. The culture vessel was left to stand in a biosafety cabinet for 15 minutes and placed in a 5% (v/v) CO₂ incubator at 37°C for 4 hours. Subsequently, the culture vessel was taken out from the incubator, and KBM ADSC-2 medium containing 1% antibiotics was additionally added. The culture vessel was returned to the 5% (v/v) CO₂ incubator at 37°C, and culture was performed for 3 days.

### Comparative Example 2

The production of spheroids was performed in the same manner as in Example 2 above, except that ELPLASIA (manufactured by Kuraray) was used instead of the cell culture vessel used in Example 2. The spheroids did not adhere to the bottom of the wells. The spheroids sank at the bottom of the wells in a non-adherent state.

### Reference Example 1

Instead of the cell culture vessel used in Example 2, a 24-well cell culture plate was used. KBM ADSC-2 medium containing 1% antibiotics was added to each well, and AdSCs were seeded on the plate and cultured in a 5% (v/v) CO₂ incubator at 37°C for 3 days.

### Test Example 3 Protein array analysis

After 3 days of culture, the culture supernatant was collected. The proteins in the supernatant were assayed using RayBio human antibody array kits (L-507, L493, RayBiotech). The measurements were performed at COSMO BIO. The obtained values were normalized to the measured value of the positive sample according to the protocol and further normalized to the number of cells (/1 × 10⁵ cells) . When the fold-change between samples was 1.5 or greater or 0.65 or smaller, the difference between the samples was considered to be significant according to the protocol.

The levels of the undifferentiation markers Nanog, Oct 3/4, SSEA-4, and SOX2 in the spheroids produced in Example 2 were significantly higher than those in the non-adherent spheroids produced in Comparative Example 2 and in the cells obtained in two-dimensional culture in Reference Example 1 (Fig. 11). More specifically, the levels of Nanog, Oct 3/4, SSEA-4, and SOX2 in the spheroids produced in Example 2 were 2.7-, 2.8-, 1.8-, and 4.7-fold higher than the corresponding levels in the spheroids produced in Comparative Example 2, respectively (Table 1). In addition, the levels of Nanog, Oct 3/4, SSEA-4, and SOX2 in the spheroids produced in Example 2 were 3.0-, 2.0-, 1.8-, and 2.4-fold higher than the corresponding levels in the cells obtained in two-dimensional culture in Reference Example 1, respectively (Table 2).

**[Table 1]**

| | Example 2 | Comparative Example 2 |
|---|---|---|
| Nanog | 2.7 | 1.0 |
| Oct 3/4 | 2.8 | 1.0 |
| SSEA-4 | 1.8 | 1.0 |
| SOX2 | 4.7 | 1.0 |

**[Table 2]**

| | Example 2 | Reference Example 1 |
|---|---|---|
| Nanog | 3.0 | 1.0 |
| Oct 3/4 | 2.0 | 1.0 |
| SSEA-4 | 1.8 | 1.0 |
| SOX2 | 2.4 | 1.0 |

### Test Example 4 Quantification of integrin αv

The cells used were the same as those used in Example 2.

### Preparation of cells

Frozen cells were thawed in a constant temperature bath at 37°C and added to 9 mL of KBM ADSC-2 medium (basal medium; manufactured by Kohjin-Bio Co., Ltd.) containing 5% FBS and 1% antibiotics. After centrifugation at 210 × g for 5 minutes, the supernatant was removed, and the cells were suspended in 1 mL of the basal medium to prepare a cell suspension.

The basal medium was added to an 800-mL cell culture flask with a filter cap (manufactured by Sumitomo Bakelite), and the cell suspension was added at a concentration of 1.0 × 10⁶ cells/flask, making a total volume of 30 mL. The flask with a filter cap was placed in a 5% (v/v) CO₂ incubator at 37°C for cell culture (expansion culture). After cell culture, the medium was removed from the flask, 5 mL of a cell detachment solution, Accutase^{™} (manufactured by PromoCell), was added, and the flask was left to stand at room temperature for about 5 minutes for cell detachment. The cell detachment solution together with the cells were recovered and transferred to a tube with the basal medium, making a total volume of 15 mL. The tube was centrifuged at 210 × g for 5 minutes, and the supernatant was removed. The remaining cells were suspended in 1 mL of KBM ADSC-2 medium containing 1% antibiotics. The number of cells was counted, and the concentration of the cell suspension was adjusted to 1 × 10⁶ cells/mL.

### Degassing

The cell culture vessel containing medium was degassed. More specifically, about 1 mL of PBS was added to the cell culture vessel, pipetting was performed, and the cell culture vessel was placed in a 5% (v/v) CO₂ incubator at 37°C for 15 minutes. Then, newly generated bubbles in the PBS were removed by pipetting, and 0.2 mL of KBM ADSC-2 medium containing 1% antibiotics was added to the cell culture vessel, which was then placed in a 5% (v/v) CO₂ incubator at 37°C overnight.

### Example 3

The cell culture vessel used was the same as that used in Example 2.

The medium was removed from the cell culture vessel subjected to degassing, and the cells were seeded at 500 cells/recess in the cell culture vessel. The cell culture vessel was left to stand in a biosafety cabinet for 15 minutes and placed in a 5% (v/v) CO₂ incubator at 37°C for 4 hours. Subsequently, the cell culture vessel was taken out from the incubator, and KBM ADSC-2 medium containing 1% antibiotics was added. The cell culture vessel was returned to the 5% (v/v) CO₂ incubator at 37°C, and culture was performed for 3 days.

The spheroids formed in the cell culture vessel were collected by pipetting on day 3 of culture. The cell extraction buffer contained in the ELISA kit (Abcam) was added to the spheroids and ice-cooled for 20 minutes to give a cell extract. The cell extract was centrifuged at 12,000 × g for 20 minutes, and the supernatant was collected. The integrin αv in the supernatant was quantified by ELISA (pg/1 × 10⁵ cells) (n = 3) .

### Comparative Example 3

The formation of spheroids and the quantification of integrin αv were performed in the same manner as in Example 3, except that the three-dimensional culture vessel ELPLASIA (Kuraray) was used as a cell culture vessel. ELPLASIA is a cell culture vessel usable for cell suspension culture and has wells made of polystyrene (depth: about 400 µm, shape of the opening: a circle with a diameter of about 500 µm, shape of the bottom: a U-shaped bottom).

### Comparative Example 4

As a cell culture vessel, a 24-well plate for two-dimensional cell culture (shape of the opening: a circle with a diameter of about 1.6 cm, shape of the bottom: a flat bottom, manufactured by Corning) was used, and the cells were seeded at 1 × 10⁵ cells/well. The cell culture vessel was left to stand in a biosafety cabinet for 15 minutes and placed in a 5% (v/v) CO₂ incubator at 37°C for 4 hours. Subsequently, the cell culture vessel was taken out from the incubator, and KBM ADSC-2 medium containing 1% antibiotics was added. The cell culture vessel was returned to the 5% (v/v) CO₂ incubator at 37°C, and culture was performed for 3 days. On day 3 of culture, 0.5 mL of Accutase^{™} was added, and the cell culture vessel was left to stand at room temperature for 5 minutes. The cells were collected by pipetting and transferred to a 1.5-mL tube (manufactured by AS ONE Corporation) . The tube was centrifuged at 510 × g for 5 minutes, and then the supernatant was discarded. The quantification of integrin αv was performed in the same manner as in Example 3.

### Results

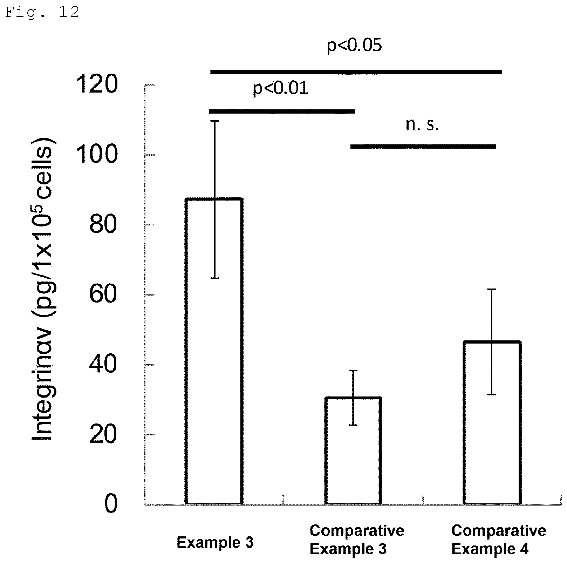

The expression level of integrin αv is shown in Fig. 12. The values refer to means ± standard deviations (n = 3), p refers to the p-value (significance probability) of the difference, and n.s. refers to not significant. The expression level of integrin αv in the spheroids of Example 3 was significantly higher than that in the spheroids of Comparative Example 3 or 4 (Fig. 12). More specifically, the expression level of integrin αv in Example 3 was 2.8-fold higher than that in Comparative Example 3 and 1.9-fold higher than that in Comparative Example 4.

### Test Example 5 Fluorescence observation of cell adhesion molecule

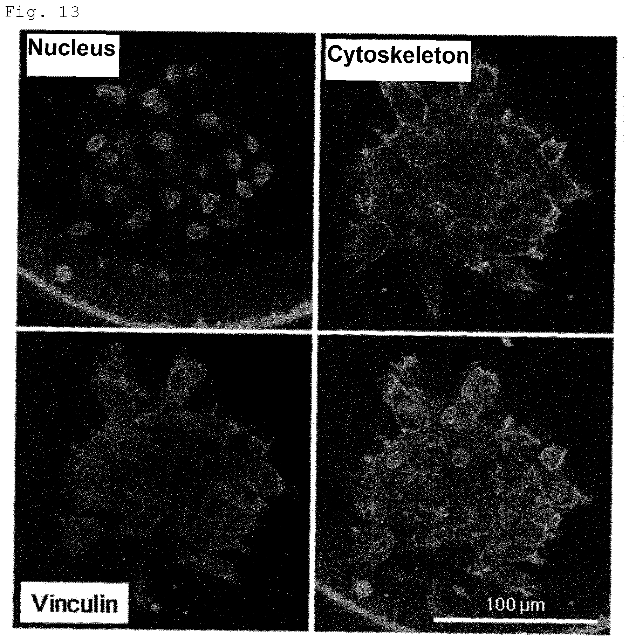

A set of fluorescence micrographs of spheroids of human adipose-derived stem cells cultured using the cell culture vessel of the present invention is shown in Fig. 13. Vinculin, one of the scaffold proteins for integrins, was remarkably expressed in the vicinity of the cytoskeleton inside the cells, indicating that integrins were highly expressed on the surface of the cell membrane and in the area close to the bottom of the recess of the cell culture sheet where the cells were attached.

### INDUSTRIAL APPLICABILITY

One aspect of the present invention enables easy preparation of undifferentiated cell spheroids and efficient cell culture operation, and therefore, the present invention is suitable for use in the field of regenerative medicine, for example. Another aspect of the present invention enables easy preparation of integrin-expressing cell spheroids, which are capable of adhering well to wound sites and unlikely to undergo cell death, and therefore, the present invention can produce integrin-expressing cell spheroids having a high potential to provide therapeutic effects in regenerative medicine, for example.

### REFERENCE SIGNS LIST

- 1: Cell Culture Sheet
- 11: Recess
- 11a: Inner circumferential face of a recess
- 11b: Bottom face of a recess
- 12: Sheet surface
- 21: Non-cell adhesive substance
- 22: Cell-adhesive substance
- 23: Adhesive layer

## Claims

1. A method for producing undifferentiated cell spheroids, the method comprising the step of culturing undifferentiated cells on a cell-adhesive surface of a cell culture sheet.

2. A method for maintaining cell spheroids in an undifferentiated state, the method comprising the step of culturing undifferentiated cell spheroids on a cell-adhesive surface of a cell culture sheet.

3. A method for producing integrin-expressing cell spheroids, the method comprising the step of culturing cells on a cell-adhesive surface of a cell culture sheet.

4. The method according to any one of claims 1 to 3, wherein the cell-adhesive surface comprises a cell-adhesive substance.

5. The method according to any one of claims 1 to 4, wherein the cell-adhesive surface comprises a polyimide resin.

6. The method according to any one of claims 1 to 5, wherein the culturing is performed on a flat cell-adhesive surface.

7. The method according to any one of claims 1 to 6, wherein the culturing is performed on a cell culture sheet having a plurality of recesses each having an opening of 2, 000 µm or less in diameter, wherein each recess has an inner circumferential face and a bottom face, wherein the inner circumferential face has a non-cell adhesive surface, and wherein the bottom face has a cell-adhesive surface.

8. The method according to any one of claims 1 to 7, wherein the culturing is performed in the absence of feeder cells.

9. The method according to any one of claims 1 to 8, wherein the cells are undifferentiated stem cells or progenitor cells.

10. The method according to claim 9, wherein the stem cells are hematopoietic stem cells, mesenchymal stem cells, neural stem cells, tissue stem cells, embryonic stem cells, or pluripotent stem cells.

11. A cell spheroid obtained by the method according to any one of claims 1 to 10.

12. An undifferentiated cell spheroid having a relative mRNA expression level of an undifferentiation marker that is at least 3-fold higher than the corresponding level in a spheroid in suspension culture on a non-cell adhesive surface, as determined per 1 × 10⁵ cells.

13. A cell spheroid having a protein expression level of an integrin that is at least 1.2-fold higher than the corresponding level in a spheroid in suspension culture on a non-cell adhesive surface.

14. The spheroid according to any one of claims 11 to 13, wherein the spheroid has a diameter of 10 to 1,500 µm.
